# EUROPEAN PATENT SPECIFICATION

(11) **EP 1 781 247 B1**
(45) Date of publication and mention of the grant of the patent: **03.11.2010**
(21) Application number: 05775939.1
(22) Date of filing: 15.08.2005
(51) Int. Cl.: A61K 8/49, A61Q 1/02

(54) **COSMETIC COMPOSITIONS CONTAINING FLUORESCENT COLORANTS FOR NATURAL SKIN APPEARANCE**
KOSMETISCHE ZUSAMMENSETZUNGEN MIT FLUORESZENTEN FARBSTOFFEN FÜR NATÜRLICHE HAUTFARBE
PRÉPARATIONS COSMÉTIQUES CONTENANT DES COLORANTS FLUORESCENTS DESTINÉS À AGIR SUR L'APPARENCE NATURELLE DE LA PEAU

(30) Priority: 23.08.2004 US 603591 P
(43) Date of publication of application: 09.05.2007
(73) Proprietor: BASF SE, 67056 Ludwigshafen (DE)
(72) Inventor: ELDER, Stewart Todd, Butler, NJ 07405 (US); MORTON, Colin, CH-4125 Riehen (CH); ANDRIANOV, Christina Ligia, Hawthorne, NJ 07506 (US)
(86) International application number: PCT/EP2005/053991
(87) International publication number: WO 2006/021527

(56) References cited:
- EP-A- 0 087 060
- EP-A- 0 370 470
- EP-A- 1 464 323
- WO-A-00/56274
- WO-A-00/71085
- WO-A-00/72808
- GB-A- 328 111
- US-A- 3 845 075
- DATABASE WPI Section Ch, Week 199133 Derwent Publications Ltd., London, GB; Class D21, AN 1991-242246 XP002353570 & JP 03 157315 A (TANAKA M) 5 July 1991 (1991-07-05)

## Description

The present invention relates to the use of fluorescent colorants in cosmetic compositions. More particularly this invention utilizes a system comprising at least one fluorescent colorant other than white in combination with at least one standard (non-fluorescent) colorant that provides unusual fluorescent effects. The final cosmetic product remains fluorescent. Cosmetic applications include all facial makeup and skin products where a natural skin appearance is desired, as well as eye products and any other personal care products that use colorants such as lipsticks.

The term fluorescent colorant embraces all fluorescent organic coloring matter and includes both pigments and dyes.

It is well known to add an optical brightener (fluorescent whitening agent) to cosmetic compositions. One recent example of this is U.S. Patent Application Publication No. 2002/0192260 A1, which discloses optically-activated particles for use in cosmetic preparations to reduce the visual perception of skin imperfections. The optically-activated particles are of various substrates such as thermoplastic polymers, natural materials, regenerated cellulose, metals and minerals which have an optical brightener chemically bonded to the substrate particles to form integral units in the form of optically-activated particles. Said particles are taught to be useful for diffusing and emitting light to reduce the visual perception of cellulite, shadows, skin discolorations and wrinkles.

However, optical brighteners only add or contribute to the blue region of the visible spectrum, which at best would give a whiter appearance to the skin.

However, it has now been found that utilizing fluorescent colors other than white, in particular those having a yellow and/or reddish hue, adds color to regions of the skin's natural color that are prominent in the skin's visible spectrum. The result is unique color effects that are unachievable by other methods.

Thus, the colorant of the invention comprises at least one fluorescent colorant other than white in addition to at least one standard (non-fluorescent) colorant. The final composition still retains fluorescent activity.

A general introduction and overview of organic colorants is provided in, W. Herbst, K. Hunger; Industrial Organic Pigments, 2nd Ed, VCH Verlagsgesellschaft, Weinheim, 1997, and a brief overview of fluorescent colorants in particular is given in B.M. Krasovitskii, B.M. Bolotin; Organic Luminescent Materials, 1988, VCH Weinheim.

A variety of colorants, from many chemical classes, show solid and/or solution state fluorescence. In principle, absorption of light energy excites molecules in these colored materials to their excited states. When deactivation of these excited states takes place via radiative decay, resulting in emission of light at a longer wavelength to the absorbed light, then fluorescence is said to have occurred. To the observer, this effect provides an unusual brilliance to the colorant, beyond that of traditional colorants employed in cosmetics. This brilliance is caused by both the ultraviolet and short-wavelength components of visible light.

Typical fluorescent colorants (both pigments and dyes), other than traditional fluorescent whitening agents, can be used in the present invention. They are materials of the following chemical classes:
a) perylene derivatives of the general formulae 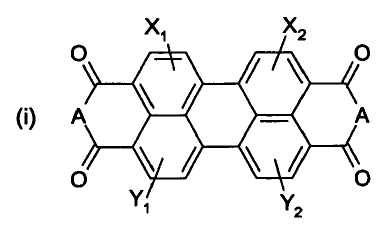 and 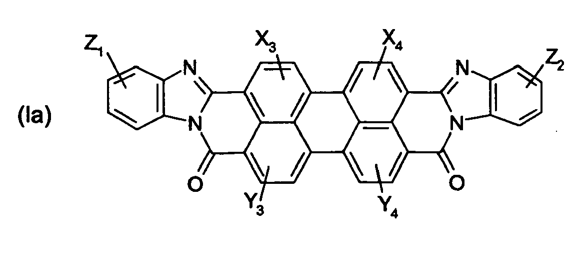 or its regioisomer, wherein
   A is O, N-H, N-alkyl or N-aryl and X₁, X₂, X₃, X₄, Y₁, Y₂, Y₃, Y₄, Z₁, Z₂ are independently halogen, O-alkyl or O-aryl, for example Pigment Red 224;
b) fluoresceins of the general formula 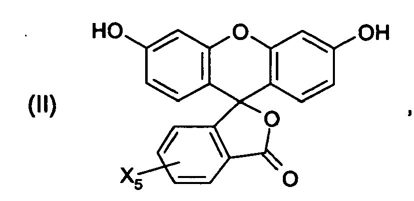 wherein
   X₅ is Cl, Br or I, for example Solvent Yellow 94, Cl 45350;
c) rhodamines of the general formula 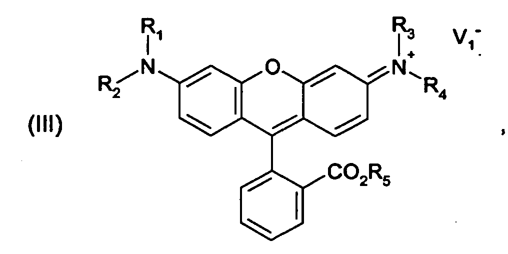 wherein
   the R₁, R₂, R₃, R₄, R₅ groups are independently H or C₁-C₁₂alkyl, and V₁ is Cl, Br or I, for example Basic Red 1, Cl 45160;
d) pyronines of the general formula 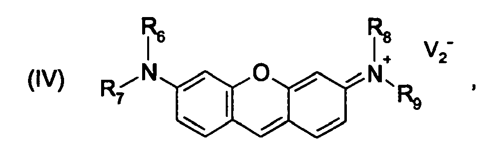 wherein R₆, R₇, R₈ and R₉ groups are independently H or C₁-C₁₂alkyl, and V₂ is Cl, Br or I, for example Pyronin G, Cl 45005;
e) naphthalic anhydride derivatives of the general formula 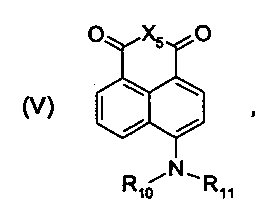 wherein
   X₅ is O, N-H, N-alkyl or N-aryl and R₁₀ and R₁₁ are independently H, alkyl or aryl, for example Solvent Yellow 43;
f) benzoxanthenes and benzthioxanthenes of the general formulae 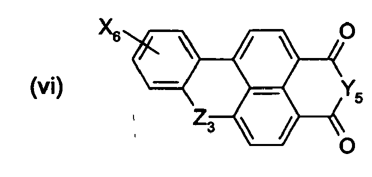 and 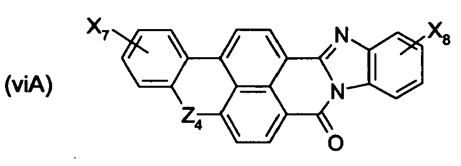 or its
   regioisomer, wherein
   X₆, X₇ and X₈ are independently O-alkyl, O-aryl or halogen,
   Y₅ is O, N-H, N-alkyl or N-aryl and Z₃ and Z₄ are independently O or S, for example Solvent Yellow 98;
g) benzanthrone derivatives of the general formula 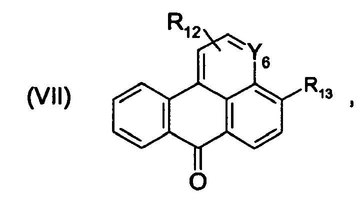 wherein
   Y₆ is C or N and R₁₂ and R₁₃ are independently halogen, alkoxy, amino or alkylamino and when
   Y₆ is C, Y₆ plus R₁₃ can form a 6-membered ring, optionally fused to an arylene group, for example Solvent Orange 63 of the formula 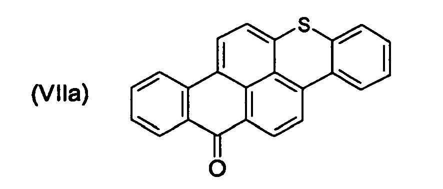
h) coumarins of the general formula 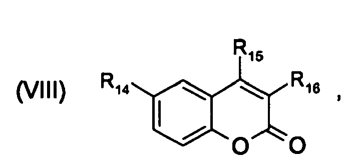 wherein
   R₁₄ is H, alkoxy, amino or alkylamino, and R₁₅ and R₁₆ are independently H, alkyl or aryl; for example solvent yellow 160;
i) isoindolo-[2,1-a]-benzimidazolone derivatives of the general formula 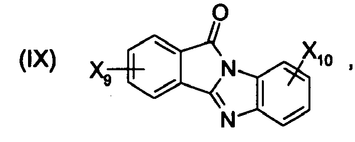 wherein
   X₉ and X₁₀ and are independently hydrogen or halogen, for example 1,2,3,4,7-pentachloro-11H-isoindolo-[2,1-a]-benzimidazol-11-one of the formula 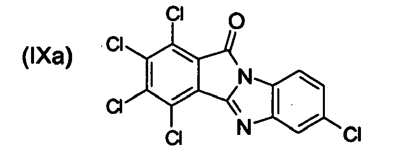
j) diphenyl maleimides of the general formula 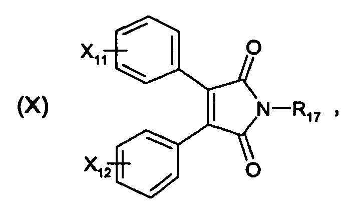 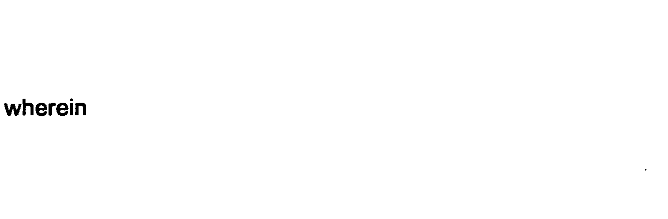 R₁₇ is optionally substituted alkyl or aryl and X₁₁ and X₁₂ are independently H, alkyl, aryl, O-alkyl, O-aryl or halogen ;
k) azo pigments of the general formula 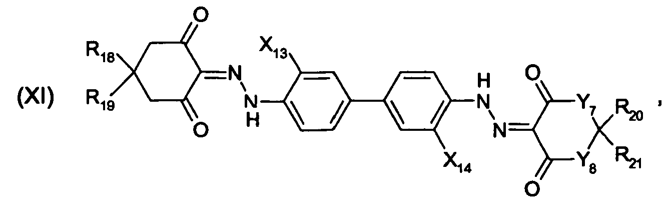 wherein
   X₁₃ and X₁₄ are independently halogen or alkoxy,
   Y₇ and Y₈ are independently CH₂ or O and
   R₁₈, R₁₉, R₂₀ and R₂₁ are independently optionally substituted alkyl or aryl;
l) diketopyrrolo[3,4-c]pyrroles (abbreviated to DPP) of the general formula 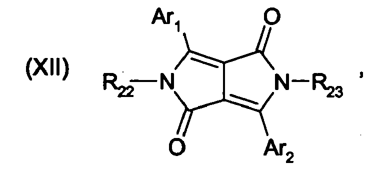 wherein
   Ar₁ and Ar₂ are aryl radicals and R₂₂ and R₂₃ are independently optionally substituted alkyl or aryl radicals.

Preferred classes of fluorescent colorants for the present invention are those having a yellow and/or reddish hue. They include perylene derivatives of the formula (I) and (Ia) such as those disclosed in DE 42 25 031, diphenyl maleimides of the formula (X) such as those disclosed in U.S. Patent No. 6,508,957, benzoxanthenes and benzthioxanthenes of the formulae (VI) and (VIa) such as those disclosed in EP 1 172 418 A2 and corresponding U.S. Patents Nos. 6,462,128 and 6,559,306 and similar compounds, N,N'-disubstituted DPPs of the formula (XII) and the isoindolo-[2,1-a]-benzimidazolone of the formula 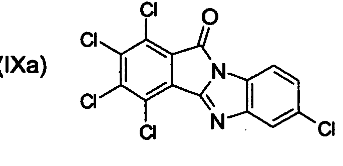 and mixtures thereof, wherein the substituents in the formulae are as defined above.

N,N'-disubstituted DPPs are disclosed or at least generically mentioned in WO 2003/022848, U.S. Patent No. 6,603,020, WO 2000/033795, U.S. Patent No. 6,372,202, U.S. Patent No. 6,042,842, WO 2004/033563 and U.S. Patent Nos. 6,113,683 and 6,160,037.

WO 2003/022848 (Ciba), relates to a new process for the preparation of certain diketopyrrolopyrroles which in particular makes it possible to obtain DPPs which are unsymmetrically substituted at the nitrogen atoms of the DPP basic unit in an acceptable yield, and to * the novel unsymmetrically substituted DPPs produced thereby. Cosmetic use is suggested but not shown, and no combination with a non-fluorescent colorant for cosmetic use is described.

U.S. Patent No. 6,603,020 (Ciba), relates to certain fluorescent DPPs of the formula 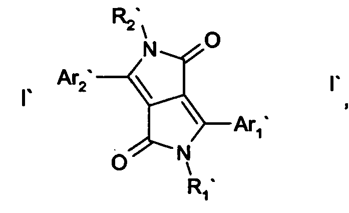 wherein
R₁' and R₂', independently from each other, stand for C₁-C₂₅-alkyl, preferably C₁-C₂₅-alkyl, which is substituted with a functional group capable of increasing the solubility in water such as a tertiary amino group, -SO₃⁻, or PO₄², allyl which can be substituted one to three times with C₁-C₃alkyl or optionally substituted phenyl or 1- or 2-naphthyl, and
Ar₁' and Ar₂', independently from each other, stand for an aryl or heteroaryl group. Cosmetic use is suggested but not shown, and no combination with a non-fluorescent colorant for cosmetic use is described.

WO 2000/033795 (Ciba), relates to cosmetic preparations or formulations for making-up the lips or the skin and for coloring the hair or the nails, comprising certain red DPP pigments of formulae 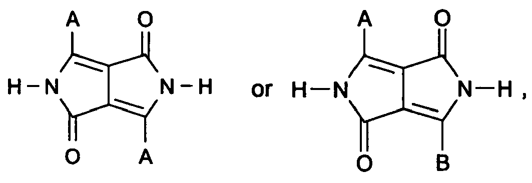 wherein
A and B are each independently of the other an aryl or heteroaryl group. Since the N-atoms are unsubstituted, the pigments have, for practical purposes, no fluorescence, i.e. they are used merely as conventional non-fluorescent red colorants.
U.S. Patent No. 6,372,202 (L'Oreal), relates to cosmetic compositions of certain special

DPPs of the formula

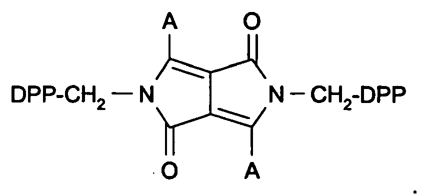

There is no teaching these DPPs are fluorescent, and no combination with a non-fluorescent colorant is shown.

U.S. Patent No. 6,042,842 (L'Oreal) relates to cosmetic compositions of a red DPP pigment with an intense and saturated color, which does not generate free radicals. While the DPPs are broadly disclosed, only Pigment Red 254 (C156110) is used. Since the N-atoms are unsubstituted in Pigment Red 254, the pigment has, for practical purposes, no fluorescence, i.e. it is used merely as a conventional non-fluorescent red colorant. Additionally, no combination with a non-fluorescent colorant is shown.

WO 2004/033563 (Ciba) relates to a method of producing colored carrier particles wherein, inter alia, certain intermediate DPPs having labile groups on the N atoms are deposited on particles, e.g. metal flakes or layered silicates, and then converted to DPPs in which the N-atoms are unsubstituted. Cosmetic use of the produced colored carrier particles, but not the fluorescent intermediates, is suggested but not shown.

U.S. Patent No. 6,113,683 discloses colored pearlescent pigments comprising a pearlescent pigment and a second pigment, and the use of an adhesion promoter for the mutual adsorbance of the pearlescent pigment and the second pigment. Since the N-atoms in the DPP pigments employed are unsubstituted, the DPP pigments have, for practical purposes, no fluorescence. Cosmetic use of the colored pearlescent pigments is suggested but not shown.

U.S. Patent No. 6,160,037 relates to mixtures containing certain pigment intermediates, inter alia DPPs, having labile groups on the N-atoms and polymerizable substances, and to a method of producing colored polymeric materials, wherein the fluorescent DPP pigment intermediates are converted via reactive extrusion into DPPs in which the N-atoms are unsubstituted. Cosmetic use of the pigment compositions is eluded to, but not shown, and no combination with a non-fluorescent colorant for cosmetic use is described.

U.S. Patent No. 6,203909 relates to a composite pigment which consists of a colorant and a substrate, using a substrate (S) and a colorant (C), which are each coated with ions or ionizable compounds having a charge rotating in the same direction, and, if desired, of additional ions or ionizable compounds having a change rotating in the same direction as a layer material (L), wherein either (I) the sign of the change of the coating of S, or the sign of the charge of the coating of S and of the charge of L, which is the same, is opposite to that of charge of the coating of C, or (II) the sign of charges of the coatings of S and C is the same and is opposite to that of the charge of L. The colorant may be a DPP pigment. Cosmetic use is suggested but not shown, and no combination with a non-fluorescent colorant for cosmetic use is described.

Particularly preferred are fluorescent colorants from the benzoxanthene class, especially the compound of the formula

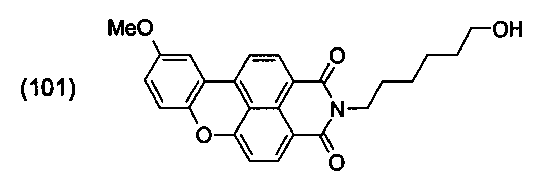

As indicated previously, the colorant of the invention comprises at least one fluorescent colorant other than white in addition to at least one standard (non-fluorescent) colorant, such that the final composition still retains fluorescent activity. The inventive colorant may additionally comprise further active ingredients, for instance UV absorbers, UV reflectors, and other cosmetically acceptable ingredients and mixture thereof.

The non-fluorescent colorant may be any colorant, for instance a dye, pigment or lake, or a mixture thereof. Typical suitable non-fluorescent colorants include any organic or inorganic pigment or colorant approved for use in cosmetics by CTFA and the FDA such as lakes, titanium dioxide (in low amounts) or other conventional pigments used in cosmetic formulations.

Examples of pigments include inorganic pigments such as carbon black, D&C Red 7, calcium lake, D&C Red 30, talc lake, D&C Red 6, barium lake, talc, kaolin, mica, mica titanium, magnesium silicate; and organic pigments such as Red No. 202, Red No. 204, Red No. 205, Red No. 206, Red No. 219, Red No. 228, Red No. 404, Yellow No. 205, Yellow No. 401, Orange No. 401 and Blue No. 404. Examples of vat dyes are Red No. 226, Blue No. 204 and Blue No. 201. Examples of lake dyes include various acid dyes which are laked with aluminum, calcium or barium.

In one embodiment the colorant is an aqueous solution of a water-soluble dye. Such dyes may include FD&C Blue No. 11, FD&C Blue No. 12, FD&C Green No. 13, FD&C Red No. 13, FD&C Red No. 140, FD&C Yellow No. 15, FD&C Yellow No. 16, D&C Blue No. 14, D&C Blue No. 19; D&C Green No. 15, D&C Green No. 16, D&C Green No. 18, D&C Orange No. 14, D&C Orange No. 15, D&C Orange No. 110, D&C Orange No. 111, D&C Orange No. 117, FD&C Red No. 14, D&C Red No. 16, D&C Red No. 17, D&C Red No. 18, D&C Red No. 19, D&C Red No. 117, D&C Red No. 119, D&C Red No. 121, D&C Red No. 122, D&C Red No. 127, D&C Red No. 128, D&C Red No. 130, D&C Red No. 131, D&C Red No. 134, D&C Red No. 139, FD&C Red No. 140, D&C Violet No. 12, D&C Yellow No. 17, Ext. D&C Yellow No. 17, D&C Yellow No. 18, D&C Yellow No. 111, D&C Brown No. 11, Ext. D&C Violet No. 12, D&C Blue No. 16 and D&C Yellow No. 110.

The above dyes are well known, commercially available materials, with their chemical structure being described, e.g., in 21 C. F. R. Part 74 (as revised April 1, 1988) and in the CTFA Cosmetic Ingredient Handbook, (1988), published by the Cosmetics, Toiletry and Fragrances Association, Inc.

The certified dyes can be water-soluble or, preferably, lakes thereof. Lakes are organic pigments prepared by precipitating a soluble dye on a reactive or absorbent stratum, which is an essential part of the pigment's composition. Most lakes are aluminum, barium or calcium derived. These insoluble pigments are used mostly in makeup products, either as powders or liquids, when a temporary color is desired that won't stain the skin (as oil-soluble dyes tend to do). The lakes are used in these products along with inorganic colors.

The following tables list currently available dyes and colorants approved for use in food, drugs and/or cosmetics. The selected colorant for use herein is preferably selected from the following exemplary lists.

| TABLE I - Dyes certified for use in foods, drugs, cosmetics (FDC colors) | | |
|---|---|---|
| FD&C Blue No. 1 | FD&C Green No. 3 | FD&C Red No. 4 |
| FD&C Red No. 40 | FD&C Yellow No. 5 | FD&C Yellow No. 6 |

| TABLE 2 - Dyes certified for topically applied drugs and cosmetics | | |
|---|---|---|
| Ext. DC Violet #2 | Ext. D&C Yellow No. 7 | Ext. D&C Violet No. 2 |
| D&C Brown No. 1 | FD&C Red No. 4 | D&C Red No. 17 |
| D&C Red No. 31 | D&C Red No. 34 | D&C Red No. 39 |
| D&C Violet No. 2 | D&C Blue No. 4 | D&C Green No. 6 |
| D&C Green No. 8 | D&C Yellow No. 7 | D&C Yellow No. 8 |
| D&C Yellow No. 11 | D&C Orange No. 4 | D&C Orange No. 10 |
| D&C Orange No. 11 | | |

| TABLE 3 - Dyes certified for drugs and foods only | | |
|---|---|---|
| D&C Blue No. 4 | D&C Brown No. 1 | D&C Green No. 5 |
| D&C Green No. 6 | D&C Green No. 8 | D&C Orange No. 4 |
| D&C Orange No. 5 | D&C Orange No. 10 | D&C Orange No. 11 |
| D&C Red No. 6 | D&C Red No. 7 | D&C Red No. 17 |
| D&C Red No. 21 | D&C Red No. 22 | D&C Red No. 27 |
| D&C Red No. 28 | D&C Red No. 30 | D&C Red No. 31 |
| D&C Red No. 33 | D&C Red No. 34 | D&C Red No. 36 |
| D&C Violet No. 2 | D&C Yellow No. 7 | D&C Yellow No. 8 |
| D&C Yellow No. 10 | D&C Yellow No. 11 | |

Some color additives are exempt from certification and permanently listed for cosmetic use, including aluminum powder, annatto, bronze powder, caramel, carmine, beta-carotene, dihydroxyacetone, disodium EDTA-copper, guanine (pearl essence), guaiazulene (azulene), mica, pyrophyllite, silver (for coloring fingernail polish), and the ultramarines (blue, green, pink, red & violet).

The cosmetic or personal care preparations and formulations according to the invention comprise from 0.005 to 20% by weight, for example 0.01 to 15%, and especially 0.10 to 10%, based on the total weight of the preparation of a mixture of at least one fluorescent colorant other than white and at least one non-fluorescent colorant. The fluorescent colorant other than white generally comprises from 0.005 to 2% by weight, for example 0.01 to 1.5%, and especially 0.5 to 1%, based on the total weight of the preparation, and the non-fluorescent colorant generally comprises from 0.005 to 20% by weight, for example 0.01 to 15%, and especially 0.10 to 10%, based on the total weight of the preparation. The non-fluorescent colorants listed on Tables 1-3 should be selected from those that will not quench the fluorescence in the cosmetic preparation. Suitable carriers for the cosmetic or personal care preparations and formulations according to the invention are the conventional cosmetically acceptable materials used in such compositions.

The cosmetic preparations and formulations according to the invention may be in the form of, for example, sticks, ointments, creams, emulsions, suspensions, dispersions, powders or solutions. They are, for example, lipsticks, mascara preparations, make-up for the cheeks, eyeshadows, foundations, eyeliners, facial and body powders or nail polishes.

If the preparations are in the form of sticks, for example lipsticks, eyeshadows, make-up for the cheeks or foundations, such preparations consist for a considerable part of fatty components, which may consist of one or more waxes, for example ozocerite, lanolin, lanolin alcohol, hydrogenated lanolin, acetylated lanolin, lanolin wax, beeswax, candelilla wax, microcrystalline wax, carnauba wax, cetyl alcohol, stearyl alcohol, cocoa butter, lanolin fatty acids, petrolatum, petroleum jelly, mono-, di- or tri-glycerides or -fatty esters that are solid at 25°C, silicone waxes, such as methyloctadecane-oxypolysiloxane and poly(dimethylsiloxy)-stearoxysiloxane, stearic acid monoethanolamine, colophane and derivatives thereof, such as glycol abietates and glycerol abietates, hydrogenated oils that are solid at 25°C, sugar glycerides and oleates, myristates, lanolates, stearates and dihydroxy stearates of calcium, magnesium, zirconium and aluminium.

The fatty component may also consist of a mixture of at least one wax and at least one oil, in which case the following oils, for example, come into consideration: paraffin oil, purcellin oil, perhydrosqualene, sweet almond oil, avocado oil, calophyllum oil, castor oil, sesame oil, jojoba oil, mineral oils having a boiling point of approximately from 310 to 410°C, silicone oils, such as dimethylpolysiloxane, linoleic alcohol, linolenic alcohol, oleyl alcohol, cereal grain oils, such as wheatgerm oil, isopropyl lanolate, isopropyl palmitate, isopropyl myristate, butyl myristate, cetyl myristate, hexadecyl stearate, butyl stearate, decyl oleate, acetyl glycerides, octanoates and decanoates of alcohols and polyalcohols, for example of glycol and glycerol, ricinoleates of alcohols and polyalcohols, for example of cetyl alcohol, isostearyl alcohol, isocetyl lanolate, isopropyl adipate, hexyl laurate and octyldodecanol.

The fatty components in such preparations in the form of sticks may generally account for up to 99.91 % by weight of the total weight of the preparation.

The cosmetic preparations and formulations according to the invention may additionally comprise further constituents, for example glycols, polyethylene glycols, polypropylene glycols, monoalkanolamides, undyed polymeric, inorganic or organic fillers, preservatives, UV filters and reflectors or other adjuvants and additives conventionally employed in cosmetics.

Such further constituents are, for example, a natural or a synthetic or a semi-synthetic di- or tri-glyceride, a mineral oil, a silicone oil, a wax, a fatty alcohol, a Guerbet alcohol or an ester thereof, a lipophilic functional cosmetic active ingredient including sunscreens, or a mixture of such substances.

Further constituents include lipophilic functional cosmeticallly active ingredients suitable for skin cosmetics, an active ingredient combination or an active ingredient extract that is an ingredient or a mixture of ingredients approved for dermal or topical application. The following may be mentioned by way of example:
- active ingredients that offer protection against sunlight (UV filters): suitable active ingredients are filter substances (sunscreens) that are able to absorb UV radiation from sunlight and convert it into heat. According to the desired action, the following light-protection agents are preferred: light-protection agents that selectively absorb sunburncausing high-energy UV radiation in the range of approximately from 280 to 315 nm (UV-B absorbers) and transmit the longer-wave range of approximately from 315 to 400 nm (UV-A range), as well as light-protection agents that absorb only the longer-wave radiation of the UV-A range of from 315 to 400 nm (UV-A absorbers).
   Suitable light-protection agents are, for example, organic UV absorbers from the class of the p-aminobenzoic acid derivatives, salicylic acid derivatives, benzophenone derivatives, dibenzoylmethane derivatives, diphenyl acrylate derivatives, benzofuran derivatives, polymeric UV absorbers comprising one or more organosilicon radicals, cinnamic acid derivatives, camphor derivatives, trianilino-s-triazine derivatives, phenylbenzimidazolesulfonic acid and salts thereof, menthyl anthranilates, benzotriazole derivatives, and/or an inorganic micropigment selected from aluminum oxide- or silicon dioxide-coated TiO₂, zinc oxide or mica;
- active ingredients providing protection against chemical and mechanical influences: these include all substances that form a barrier between the skin and external harmful substances, for example paraffin oils, silicone oils, vegetable oils, PCL products and lanolin for protection against aqueous solutions, film-forming agents, such as sodium alginate, triethanolamine alginate, polyacrylates, polyvinyl alcohol or cellulose ethers against the effect of organic solvents, or substances based on mineral oils, vegetable oils or silicone oils as "lubricants" against severe mechanical stresses on the skin;
- moisturizing substances: the following substances, for example, are used as moisture-controlling agents (moisturizers): sodium lactate, urea, alcohols, sorbitol, glycerol, propylene glycol, collagen, elastin or hyaluronic acid;
- active ingredients having a keratoplastic effect: benzoyl peroxide, retinoic acid, colloidal sulfur and resorcinol;
- antimicrobial agents, for example triclosan or quaternary ammonium compounds;
- oily or oil-soluble vitamins or vitamin derivatives that can be applied dermally: for example vitamin A (retinol in the form of the free acid or derivatives thereof), panthenol, pantothenic acid, folic acid, and combinations thereof, vitamin E (tocopherol), F; essential fatty acids; or niacinamide (nicotinic acid amide);
- vitamin-based placenta extracts: active ingredient compositions comprising especially vitamins A, C, E, B₂₁, B₁₂, folic acid and biotin, amino acids and enzymes as well as compounds of the trace elements magnesium, silicon, phosphorus, calcium, manganese or copper;
- skin repair complexes: obtainable from inactivated and disintegrated cultures of bacteria of the bifidus group;
- plants and plant extracts: for example arnica, aloe, beard lichen, ivy, stinging nettle, ginseng, henna, camomile, marigold, rosemary, sage, horsetail or thyme;
- animal extracts: for example royal jelly, propolis, proteins or thymus extracts;
- cosmetic oils that can be applied dermally: neutral oils of the Miglyol 812 type, apricot kernel oil, avocado oil, babassu oil, cottonseed oil, borage oil, thistle oil, groundnut oil, gamma-oryzanol, rosehip-seed oil, hemp oil, hazelnut oil, blackcurrant-seed oil, jojoba oil, cherry-stone oil, salmon oil, linseed oil, cornseed oil, macadamia nut oil, almond oil, evening primrose oil, mink oil, olive oil, pecan nut oil, peach kernel oil, pistachio nut oil, rape oil, rice-seed oil, castor oil, safflower oil, sesame oil, soybean oil, sunflower oil, tea tree oil, rapeseed oil or wheatgerm oil.

The preparations in stick form are preferably anhydrous but may in certain cases comprise a certain amount of water which, however, in general does not exceed 40 % by weight, based on the total weight of the cosmetic preparation.

If the cosmetic preparations and formulations according to the invention are in the form of semi-solid products, that is to say in the form of ointments or creams, they may likewise be anhydrous or aqueous. Such preparations and formulations are, for example, mascaras, eyeliners, foundations, make-up for the cheeks, eyeshadows, or compositions for treating rings under the eyes.

If, on the other hand, such ointments or creams are aqueous, they are especially emulsions of the water-in-oil type or of the oil-in-water type that comprise, apart from the pigment, from 1 to 98.8 % by weight of the fatty phase, from 1 to 98.8 % by weight of the aqueous phase and from 0.2 to 30 % by weight of an emulsifier.

Such ointments and creams may also comprise further conventional cosmetic additives, for example perfumes, antioxidants, preservatives, gel-forming agents, UV filters, pearlescent agents, undyed polymers as well as inorganic or organic fillers.

If the preparations are in the form of a powder, they consist essentially of a mineral or inorganic or organic filler, for example talcum, kaolin, starch, polyethylene powder or polyamide powder, as well as adjuvants such as binders, etc..

Such preparations may likewise comprise various adjuvants conventionally employed in cosmetics, such as perfumes, antioxidants, preservatives, etc.

If the cosmetic preparations and formulations according to the invention are nail varnishes, they consist essentially of nitrocellulose and a natural or synthetic polymer in the form of a solution in a solvent system, it being possible for the solution to comprise other adjuvants, for example pearlescent agents.

In that embodiment, the inventive colorant mixture is present in an amount of approximately from 0.1 to 5 % by weight.

The cosmetic preparations and formulations according to the invention are prepared in the conventional manner, for example by mixing or stirring the components together, optionally with heating, so that the mixtures melt.

In one embodiment the present invention also includes a method of coloring a cosmetic or personal care preparation, which comprises incorporating therein a tinctorially effective amount of a composition comprising at least one fluorescent colorant other than white and at least one non-fluorescent colorant.

In a preferred embodiment of the method the fluorescent colorant is selected from the group consisting of condensation products of p-N,N'-di(methyl)aminobenzaldehyde and barbituric acid, the benzoxanthene of the formula (101)
and mixtures thereof.

The present invention also includes a method of coloring human skin, which comprises applying to said skin a cosmetic or personal care preparation which comprises a tinctorially effective amount of at least one fluorescent colorant other than white and at least one non-fluorescent colorant.

In a preferred embodiment of this method the fluorescent colorant is selected from the group consisting of condensation products of p-N,N'-di(methyl)aminobenzaldehyde and barbituric acid, the benzoxanthene of the formula (101) and mixtures thereof.

The following examples describe certain embodiments of this invention, but the invention is not limited thereto. It should be understood that numerous changes to the disclosed embodiments can be made in accordance with the disclosure herein without departing from the or scope of the invention. These examples are therefore not meant to limit the scope of the invention. Rather, the scope of the invention is to be determined only by the appended claims. In these examples all parts given are by weight unless otherwise indicated.

### Example 1.

| Lipstick with Fluorescent Organic Pigment | | | | |
|---|---|---|---|---|
| Phase | INCI Name | Trade Name | Supplier | % |
| A | Castor Oil | Lipovol CO | Lipo | 45.10 |
| A | Octyldodecyl Stearoyl Stearate | Dermol 20-SS | Alzo | 12.90 |
| A | Carprylic/Capric Triglycerides | Pelemol CCT | Phoenix | 6.60 |
| A | Octyl Isononoate | Pelemol 89 | Phoenix | 2.70 |
| A | Mineral Oil | Mineral Oil | Univ. Preserve a chem | 1.30 |
| A | Euphoria Cerifera | Candelilla Wax SP 75 | S&P | 6.50 |
| A | White Beeswax | White Beeswax | S&P | 3.90 |
| A | Carnauba Wax | Carnauba Wax SP63 | S&P | 0.70 |
| A | Ozokerite | Ozokerite | S&P | 2.90 |
| A | Methyl Glucose Sesquistearate | Glucate SS | Amerchol | 1.90 |
| A | Dodecyl Laurate, Lauryl Laurate | Purester-24 | S&P | 2.80 |
| **B** | Hydrogenated Polydecene | Silkflo 366NF | Lipo | 0.70 |
| B | Mica | 280 Mica Cosmetic, BC | Whittacker | 9.00 |
| B | | Fluorescent organic | Ciba Specialty | |
| | Compound A* | colorant | Chemicals | 3.00 |
| | | | Total | 100.00 |

| | | | | |
|---|---|---|---|---|
| * benzoxanthene of formula (101). | | | | |

### Procedure:

All the ingredients (Phase A) are melted together in a beaker at 80-85°C using a propeller mixer until a uniform waxy base is formed. In a separate beaker, the ingredients of Phase B are combined and thoroughly mixed. The temperature of Phase A is lowered to about 72-75°C and then Phase B is added with thorough mixing. The lipstick is poured into molds at 72-75°C and then allowed to cool to room temperature.

### Example 2.

| 1. O/W Facial Foundation with Fluorescent Pigments | | | | |
|---|---|---|---|---|
| Phase | INCI Name | Trade Name | Supplier | % |
| A | DI Water | DI Water | N/A | 68.72 |
| | TEA 99% | TEA 99% | | 0.20 |
| | PEG-12 Dimethicone | DC 193 | Dow | 0.10 |
| B | Butylene Glycol | Jeechem BUGL | Jeen | 4.00 |
| | Magnesium Aluminum Silicate | Veegum Granules OD-252 | R.T. Vanderbilt | 0.50 |
| | | | | |
| C | Butylene Glycol | Butylene Glycol | Jeen | 2.00 |
| | Cellulose | Sodium Carboxymethylcellulose | Hercules | 0.10 |
| D | Methylparaben | Nipagin M | Clariant | 0.10 |
| E | Compound A* | Fluorescent organic colorant | Ciba Specialty Chemicals | 0.11 |
| | Condensate B** | barbituric acid condensate | Ciba Specialty Chemicals | 0.30 |
| | Encap. Red 7 Ca Lake | Encap. Red 7 Ca Lake | Ciba Specialty Chemicals | 0.38 |
| | Encap. Blue 1 Al Lake Di-PPG-3 | Encap. Blue 1 Al Lake | Ciba Specialty Chemicals | 0.09 |
| F | Myristyl Ether Adipate | Promyristyl PM3 | Croda | 12.00 |
| | Dioctyl Maleate | Pelemol DOM | Phoenix Chemicals | 3.00 |
| | Steareth-10 | Lipocol S-10 | Lipo | 1.00 |
| | Steareth-2 | Lipocol S-2 | Lipo | 0.50 |
| | Cetyl Alcohol | Lipocol C | Lipo | 0.60 |
| | Crodafos CES | Crodafos CES | Croda | 4.00 |
| | Propyl Paraben | Nipasol M | Clariant | 0.10 |
| G | Talc | Altalc 400V | Whittacker | 2.00 |
| H | DMDM Hydantoin | Nipaguard DMDMH | Clariant | 0.20 |
| | | | Total | 100.00 |

| | | | | |
|---|---|---|---|---|
| * benzoxanthene of formula (101) ** condensation products of p-N,N'-di(methyl)aminobenzaldehyde and barbituric acid having the CAS numbers 1753-475 and 152734-34-4. | | | | |

### Procedure:

In a suitably sized beaker phase A is weighed out and mixed with a homogenizer for 5 minutes. Then heating the mixture is begun. Phase B is added to A and the mixture is homogenized for 30-40 minutes at 75-80°C. Phase C is added to the beaker and the mixture is homogenized for another 30 minutes at 75-80°C. Phase D is added and the mixture is mixed for 5 minutes. Phase E is added and the mixture is homogenized until the pigments are completely dispersed and no particles are seen floating throughout. In a separate beaker, phase F is melted at 75-80°C and mixed well until a uniform liquid solution is obtained, which is then added to the heated mixture. The mixture is homogenized for 15 minutes at 75-80°C; then cooling the mixture is begun. Phase G is added at 70°C and the mixture is homogenized for 5-10 minutes, followed by paddle mixing. At 45°C or below phase H is added. Slow mixing is stopped when the mixture reaches room temperature.

### Example 3: (Comparative example showing loss of fluorescence with the addition of iron oxide colorants)

| O/W Facial Foundation with Fluorescent Pigments and Iron Oxides | | | | |
|---|---|---|---|---|
| Phase | INCI Name | Trade Name | Supplier | % |
| A | DI Water | DI Water | N/A | 69.30 |
| | TEA 99% | TEA 99% | | 0.20 |
| | PEG-12 Dimethicone | DC 193 | Dow | 0.10 |
| | | | | |
| B | Butylene Glycol | Jeechem BUGL | Jeen | |
| | Magnesium Aluminum Silicate | Veegum Granules OD-252 | R.T. Vanderbilt | 0.50 |
| C | Butylene Glycol | Butylene Glycol | Jeen | 2.00 |
| | Cellulose | Sodium Carboxymethylcellulose | Hercules | 0.10 |
| D | Methylparaben | Nipagin M | Clariant | 0.10 |
| E | Compound A* | Fluorescent organic colorant | Ciba Specialty Chem. | 0.10 |
| | Condensate B** | barbituric acid condensate | Ciba Specialty Chem. | 0.10 |
| | Black Iron Oxide | C33-5198 | Sun Chemicals | 0.10 |
| F | Di-PPG-3 Myristyl Ether Adipate | Promyristyl PM3 | Croda | 12.00 |
| | Dioctyl Maleate | Pelemol DOM | Phoenix Chemicals | 3.00 |
| | Steareth-10 | Lipocol S-10 | Lipo | 1.00 |
| | Steareth-2 | Lipocol S-2 | Lipo | 0.50 |
| | Cetyl Alcohol | Lipocol C | Lipo | 0.60 |
| | Crodafos CES | Crodafos CES | Croda | 4.00 |
| | Propyl Paraben | Nipasol M | Clariant | 0.10 |
| G | Talc | Altalc 400V | Whittacker | 2.00 |
| H | DMDM Hydantoin | Nipaguard DMDMH | Clariant | 0.20 |
| | | | Total | 100.00 |

| | | | | |
|---|---|---|---|---|
| * benzoxanthene of formula (101) ** condensation products of p-N,N'-di(methyl)aminobenzaldehyde and barbituric acid having the CAS numbers 1753-475 and 152734-34-4. | | | | |

### Procedure:

In a suitably sized beaker phase A is weighed out and mixed with a homogenizer for 5 minutes. Then heating the mixture is started. Phase B is added to A and the mixture is homogenized for 30-40 minutes at 75-80°C. Phase C is added to the beaker and the mixture is homogenized for another 30 min. at 75-80°C. Phase D is added and the mixture is mixed for 5 minutes. Phase E is added and the mixture is homogenized until the pigments are completely dispersed and no particles are seen floating throughout In a separate beaker, phase F is melted at 75-80°C and mixed well until a uniform liquid solution is obtained, which is then added to the heated mixture. The mixture is homogenized for 15 minutes at 75-80°C; then cooling the mixture is begun. Phase G is added at 70°C and the mixture is homogenized for 5-10 minutes. Then paddle mixing and cooling is begun. Phase H is added at 45°C or below. Slow mixing is stopped when the mixture reaches room temperature.

The resulting facial foundation gives a colored preparation that is devoid of fluorescence due to the presence of iron oxide.

### Example 4. Pressed Eyeshadow with Fluorescent Pigments

| Phase | INCI Name | Trade Name | Supplier | % |
|---|---|---|---|---|
| A | Mica | Sericite PHN | Presperse | 75.60 |
| A | Zinc Stearate | Zinc Stearate | Witco | 5.00 |
| A | Condensate B** | barbituric acid condensate | Ciba Specialty Chemicals | 6.00 |
| A | Compound A* | Fluorescent organic colorant | Ciba Specialty Chemicals | 2.00 |
| A | Methylparaben | Nipagin M | Clariant | 0.20 |
| A | Propylparaben | Nipasol M | Clariant | 0.10 |
| A | Calcium Aluminum Borosilicate | Luxil | Presperse | 5.00 |
| A | PEG-4 Diheptanoate | Liponate 2-DH | Lipo | 5.50 |
| | | | Total | 100.00 |

| | | | | |
|---|---|---|---|---|
| * benzoxanthene of formula (101) ** condensation products of p-N,N'-di(methyl)aminobenzaldehyde and barbituric acid having the CAS numbers 1753-475 and 152734-34-4. | | | | |

### Procedure:

The ingredients are combined and mixed well; then heated to 100°C and pressed at 2000 psi.

### Example 5: Preparation of fluorescent organic colorant of formula (101):

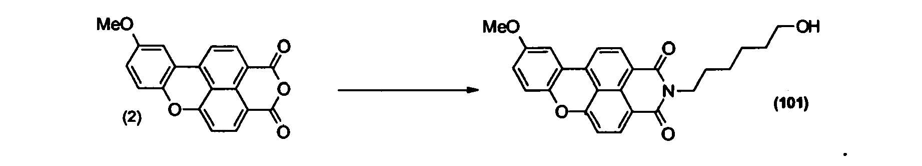

A mixture of the methoxybenzoxanthene derivative 2 [prepared according to literature methods disclosed in US 3,741,971 to O. Fuchs et al.] (5 g, 0.0157 mol), 6-amino-1-hexanol (2.12 g, 0.0181 mol), toluene sulfonic acid monohydrate (0.21 g, 0.0011 mol) and N,N-dimethylformamide (200 ml) was stirred with heating to 135°C under nitrogen for 18 hours, then cooled to 25°C. Methanol (20 ml) was then added, the mixture stirred for 30 minutes, then the yellow precipitate was filtered off, washed with methanol and then water and dried *in vacuo* at 40°C. Yield = 4.88 g, 77%.

## Claims

1. A cosmetic or personal care composition having fluorescent activity, which comprises at least one fluorescent colorant selected from the group consisting of
a) perylene derivatives of the general formulae 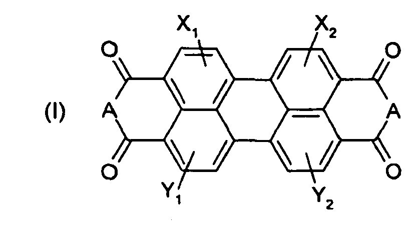 and 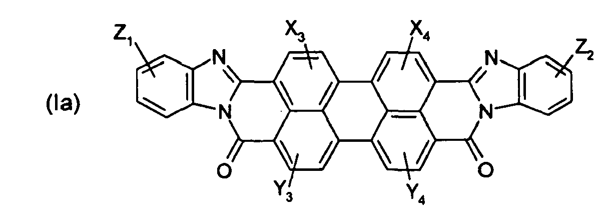 or its regioisomer, wherein
A is O, N-H, N-alkyl or N-aryl and X₁, X₂, X₃, X₄, Y₁, Y₂, Y₃, Y₄, Z₁, Z₂ are independently halogen, O-alkyl or O-aryl;
b) fluoresceins of the general formula 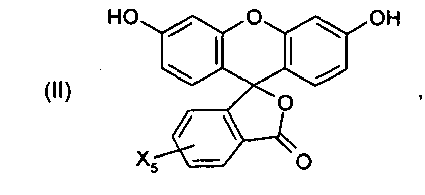 wherein
X₅ is Cl, Br or I;
c) rhodamines of the general formula 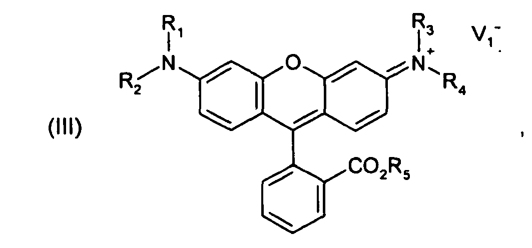 wherein
the R₁, R₂, R₃, R₄, R₅ groups are independently H or C₁-C₁₂alkyl, and V₁ is Cl, Br or I;
d) pyronines of the general formula 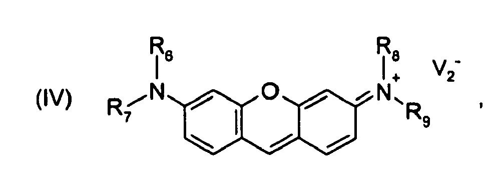 wherein
R₆, R₇, R₈ and R₉ groups are independently H or C₁-C₁₂alkyl, and V₂ is Cl, Br or I;
e) naphthalic anhydride derivatives of the general formula 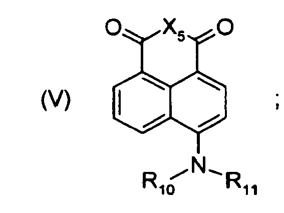 wherein
X₅ is O, N-H, N-alkyl or N-aryl and R₁₀ and R₁₁ are independently H, alkyl or aryl;
f) benzoxanthenes and benzthioxanthenes of the general formulae 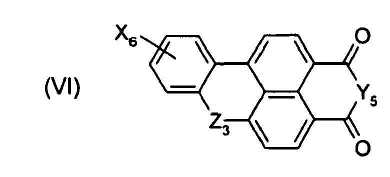 and 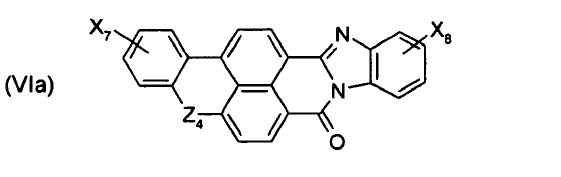 or its regioisomer, wherein
X₆, X₇ and X₈ are independently O-alkyl, O-aryl or halogen,
Y₅ is O, N-H, N-alkyl or N-aryl and Z₃ and Z₄ are independently O or S;
g) benzanthrone derivatives of the general formula 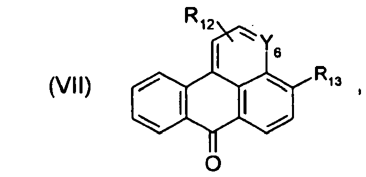 wherein
Y₆ is C or N and R₁₂ and R₁₃ are independently halogen, alkoxy, amino or alkylamino and
when
Y₆ is C, Y₆ plus R₁₃ can form a 6-membered ring, optionally fused to an arylene group;
h) coumarins of the general formula 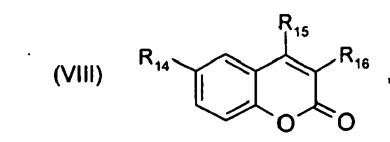 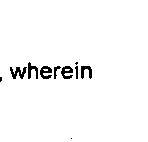 R₁₄ is H, alkoxy, amino or alkylamino, and R₁₅ and R₁₆ are independently H, alkyl or aryl;
i) isoindolo-[2,1-a]-benzimidazolone derivatives of the general formula 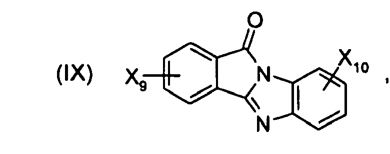 wherein
X₉ and X₁₀ and are independently hydrogen or halogen;
j) diphenyl maleimides of the general formula 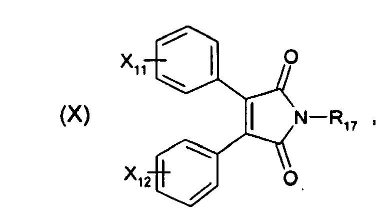 wherein
R₁₇ is optionally substituted alkyl or aryl and X₁₁ and X ₁₂ are independently H, alkyl, aryl, O-alkyl, O-aryl or halogen ;
k) azo pigments of the general formula 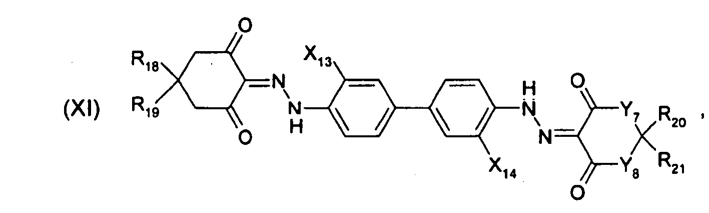 wherein
X₁₃ and X₁₄ are independently halogen, alkoxy, Y₇ and Y₈ are independently CH₂ or O and
R₁₈, R₁₉, R₂₀ and R₂₁ are independently optionally substituted alkyl or aryl;
l) diketopyrrolo[3,4-c]pyrroles of the general formula 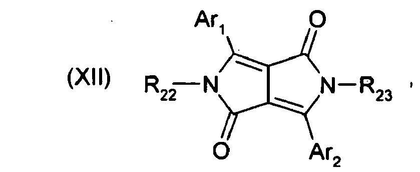 wherein
Ar₁ and Ar₂ are aryl radicals and R₂₂ and R₂₃ are optionally substituted alkyl or aryl radicals and mixtures thereof and at least one non-fluorescent colorant.

2. A composition according to claim 1, which provides a natural skin appearance.

3. A composition according to claim 1, wherein the fluorescent colorant has a yellow and/or reddish hue.

4. A composition according to claim 1, wherein the fluorescent colorant is selected from the group consisting of perylene derivatives of the formula (I) or (Ia), diphenyl maleimides of the formula (X), benzoxanthenes and benzthioxanthenes of the formulae (VI) and (VIa), N,N'-disubstituted DPPs of the formula (XII) and the isoindolo-[2,1-a]-benzimidazolone of the formula 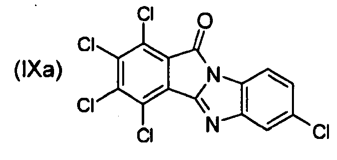 and mixtures thereof, wherein the substituents in the formulae are as defined in claim 1.

5. A composition according to claim 1, wherein the fluorescent colorant is the benzoxanthene of the formula 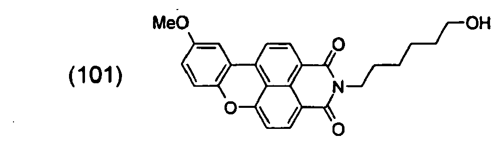 and mixtures thereof.

6. A composition according to claim 1, wherein the non-fluorescent colorant is a dye, pigment or lake, or a mixture thereof.

7. A cosmetic or personal care preparation or formulation having fluorescent activity, which comprises from 0.005 to 20% by weight, based on the total weight of the preparation of a mixture of at least one fluorescent colorant according to claims 1 to 5 and at least one non-fluorescent colorant and a cosmetically acceptable adjuvant.

8. A preparation according to claim 7, which comprises 0.005 to 2% by weight of the fluorescent colorant.

9. A preparation according to claim 7, which comprises 0.005 to 20% by weight of the non-fluorescent colorant.

10. A preparation according to claim 7, which is in the form of a lipstick, mascara preparation, make-up for the cheeks, eyeshadow, foundation, eyeliner, facial or body powder or nail polish.

11. A preparation according to claim 7, which comprises additional cosmetically acceptable active ingredients.

12. A preparation according to claim 7, wherein the cosmetically acceptable active ingredients are glycols, polyethylene glycols, polypropylene glycols, monoalkanolamides, undyed polymeric, inorganic or organic fillers, preservatives, UV filters and reflectors or other adjuvants and additives conventionally employed in cosmetics.

13. A method of coloring a cosmetic or personal care preparation, which comprises incorporating therein a tinctorially effective amount of a mixture of at least one fluorescent colorant according to claims 1 to 5 and at least one non-fluorescent colorant according to claim 1.

14. A method according to claim 13, wherein the fluorescent colorant is the benzoxanthene of the formula (101) and mixtures thereof.

15. A method of coloring human skin, which comprises applying to said skin a cosmetic or personal care preparation according to claim 7.

16. A method according to claim 15, wherein the fluorescent colorant is the benzoxanthene of the formula (101) and mixtures thereof.

17. A method of preparing a colored cosmetic or personal care preparation according to claim 7, which comprises incorporating into said preparation a tinctorially effective amount of a mixture of at least one fluorescent colorant according to claims 1 to 5 and at least one non-fluorescent colorant.

18. The benzoxanthene of the formula

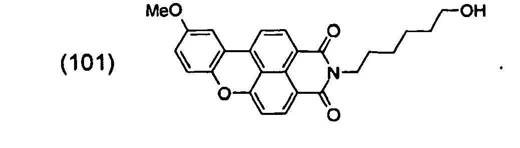

## Patentansprüche

1. Kosmetische oder Körperpflegezusammensetzung mit fluoreszierender Wirkung, umfassend wenigstens einen Fluoreszenzfarbstoff, ausgewählt aus der Gruppe, bestehend aus
a) Perylenderivaten der allgemeinen Formeln 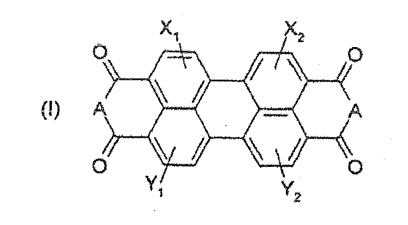 und 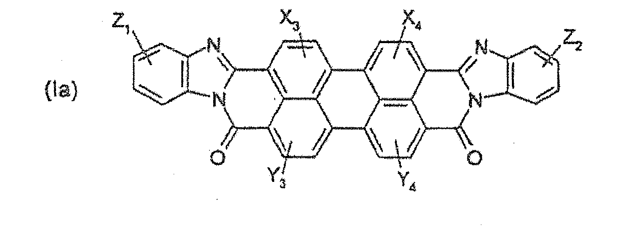 oder dessen Regioisomer, wobei
A 0, N-H, N-Alkyl oder N-Aryl ist, und X₁, X₂, X₃, X₄, Y₁, Y₂, Y₃, Y₄, Z₁, Z₂ unabhängig Halogen, 0-Alkyl oder 0-Aryl sind;
b) Fluoresceinen der allgemeinen Formel 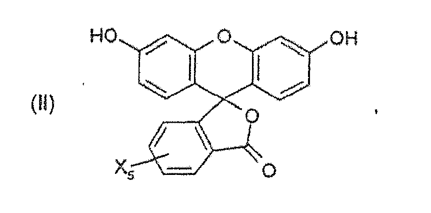 wobei
X₅ Cl, Br oder I ist;
c) Rhodaminen der allgemeinen Formel 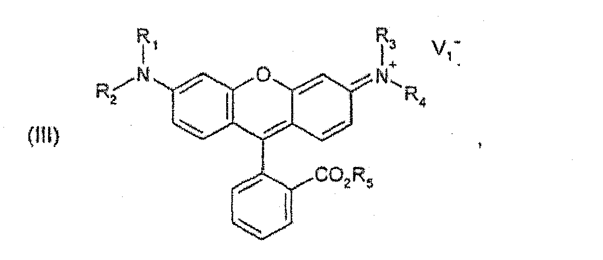 wobei
die Reste R₁, R₂, R₃, R₄, R₅ unabhängig H oder C₁-C₁₂-Alkyl sind, und
V₁ Cl, Br oder I ist;
d) Pyroninen der allgemeinen Formel 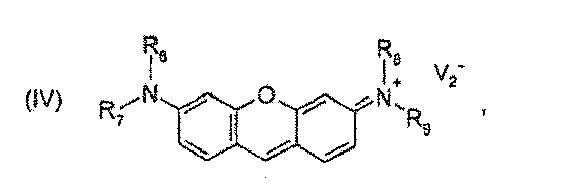 wobei
die Reste R₆, R₇, R₈ und R₉ unabhängig H oder C₁-C₁₂-Alkyl sind, und
V₂ Cl, Br oder I ist;
e) Naphthalinsäureanhydridderivaten der allgemeinen Formel 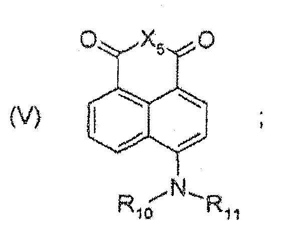 wobei
X₅ O, N-H, N-Alkyl oder N-Aryl ist, und R₁₀ und R₁₁ unabhängig H, Alkyl oder Aryl sind;
f) Benzoxanthenen und Benzthioxanthenen der allgemeinen Formeln 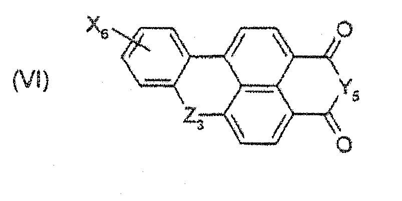 und 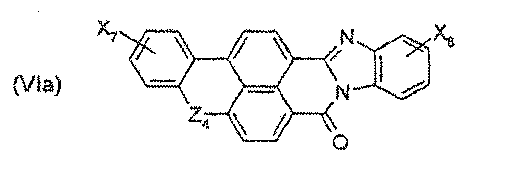 oder dessen Regioisomer, wobei
X₆, X₇ und X₈ unabhängig 0-Alkyl, 0-Aryl oder Halogen sind,
Y₅ O, N-H, N-Alkyl oder N-Aryl ist und Z₃ und Z₄ unabhängig 0 oder S sind;
g) Benzanthronderivaten der allgemeinen Formel 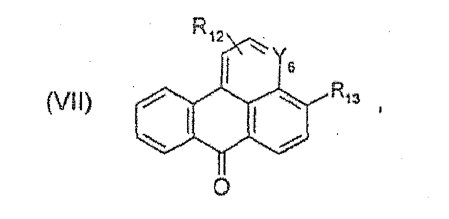 wobei
Y₆ C oder N ist, und R₁₂ und R₁₃ unabhängig Halogen, Alkoxy, Amino oder Alkylamino sind, und wenn Y₆ C ist, Y₆ plus R₁₃ einen 6-gliedrigen Ring bilden können, gegebenenfalls mit einem Arylenrest verschmolzen;
h) Cumarinen der allgemeinen Formel 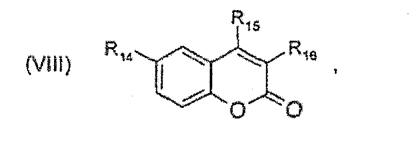 wobei
R₁₄ H, Alkoxy, Amino oder Alkylamino ist, und R₁₅ und R₁₆ unabhängig H, Alkyl oder Aryl sind;
i) Isoindolo-[2,1-a]benzimidazolonderivaten der allgemeinen Formel 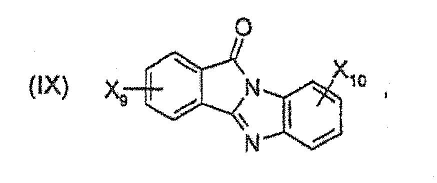 wobei
X₉ und X₁₀ unabhängig Wasserstoff oder Halogen sind;
j) Diphenylmaleimiden der allgemeinen Formel 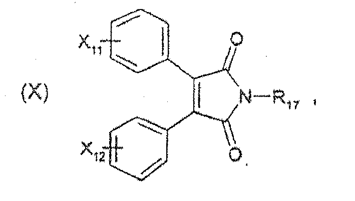 wobei
R₁₇ gegebenenfalls substituiertes Alkyl oder Aryl ist, und X₁₁ und X₁₂ unabhängig H, Alkyl, Aryl, 0-Alkyl, O-Aryl oder Halogen sind;
k) Azopigmenten der allgemeinen Formel 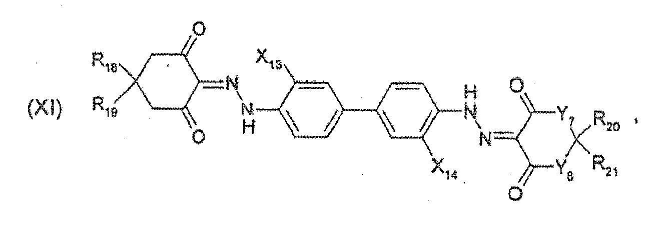 wobei
X₁₃ und X₁₄ unabhängig Halogen, Alkoxy sind, Y₇ und Y₈ unabhängig CH₂ oder 0 sind, und R₁₈, R₁₉, R₂₀ und R₂₁ unabhängig gegebenenfalls substituiertes Alkyl oder Aryl sind;
l) Diketopyrrolo[3,4-c]pyrrolen der allgemeinen Formel 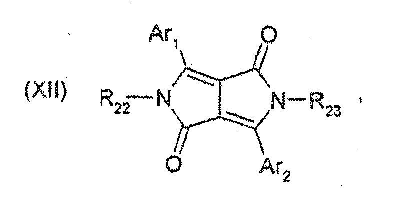 wobei
Ar₁ und Ar₂ Arylreste sind, und R₂₂ und R₂₃ gegebenenfalls substituierte Alkyl- oder Arylreste sind,
sowie Gemische davon mit wenigstens einem nichtfluoreszierenden Farbmittel.

2. Zusammensetzung gemäß Anspruch 1, die ein natürliches Aussehen der Haut bereitstellt.

3. Zusammensetzung gemäß Anspruch 1, wobei der Fluoreszenzfarbstoff einen gelben und/oder rötlichen Farbton aufweist.

4. Zusammensetzung gemäß Anspruch 1, wobei der Fluoreszenzfarbstoff ausgewählt ist aus der Gruppe, bestehend aus Perylenderivaten der Formeln (I) oder (Ia), Diphenylmaleimiden der Formel (X), Benzoxanthenen und Benzthioxanthenen der Formeln (VI) und (VIa), N,N'-disubstituierten DPPs der Formel (XII) und dem Isoindolo-[2,1-a]benzimidazolon der Formel 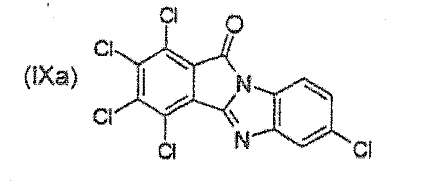 und Gemischen davon, wobei die Substituenten in den Formeln wie in Anspruch 1 definiert sind.

5. Zusammensetzung gemäß Anspruch 1, wobei der Fluoreszenzfarbstoff das Benzoxanthen der Formel 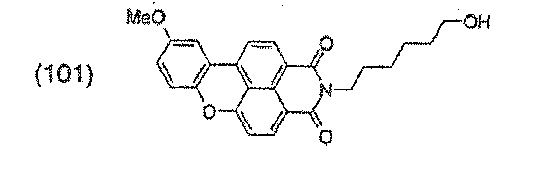 ist und Gemische davon.

6. Zusammensetzung gemäß Anspruch 1, wobei das nichtfluoreszierende Farbmittel ein Farbstoff, Pigment oder Lack ist, oder ein Gemisch davon.

7. Kosmetische(s) oder Körperpflegepräparat oder -formulierung mit fluoreszierender Wirkung, umfassend 0,005 bis 20 Gew.-%, bezogen auf das Gesamtgewicht des Präparats, eines Gemischs von wenigstens einem Fluoreszenzfarbstoff gemäß Ansprüchen 1 bis 5 und wenigstens einem nichtfluoreszierenden Farbmittel, sowie einen kosmetisch verträglichen Hilfsstoff.

8. Präparat gemäß Anspruch 7, umfassend 0,005 bis 2 Gew.-% des Fluoreszenzfarbstoffs.

9. Präparat gemäß Anspruch 7, umfassend 0,005 bis 20 Gew.-% des nichtfluoreszierenden Farbmittels.

10. Präparat gemäß Anspruch 7, das in der Form eines Lippenstifts, eines Maskarapräparats, eine Makeups für die Wangen, Lidschatten, Grundlage, Eyeliner, Gesichts- oder Körperpulver oder Nagellack vorliegt.

11. Präparat gemäß Anspruch 7, das zusätzliche kosmetisch verträgliche Wirkstoffe umfasst.

12. Präparat gemäß Anspruch 7, wobei die kosmetisch verträglichen Wirkstoffe Glycole, Polyethylenglycole, Polypropylenglycole, Monoalkanolamide, nichtgefärbte polymere, anorganische oder organische Füllstoffe, Konservierungsmittel, UV-Filter und Reflektoren oder andere Hilfsstoffe und Zusatzstoffe sind, die herkömmlich in Kosmetika eingesetzt werden.

13. Verfahren zum Färben eines kosmetischen oder Körperpflegepräparats, umfassend das Einverleiben darin einer färbewirksamen Menge eines Gemischs von wenigstens einem Fluoreszenzfarbstoff gemäß Ansprüchen 1 bis 5 und wenigstens einem nichtfluoreszierenden Farbmittel gemäß Anspruch 1.

14. Verfahren gemäß Anspruch 13, wobei der Fluoreszenzfarbstoff das Benzoxanthen der Formel (101) ist sowie Gemische davon.

15. Verfahren zum Färben menschlicher Haut, umfassend das Auftragen eines Kosmetikums oder Körperpflegepräparats gemäß Anspruch 7 auf die Haut.

16. Verfahren gemäß Anspruch 15, wobei der Fluoreszenzfarbstoff das Benzoxanthen der Formel (101) ist sowie Gemische davon.

17. Verfahren zum Herstellen eines gefärbten kosmetischen oder Körperpflegepräparats gemäß Anspruch 7, umfassend das Einverleiben einer färbewirksamen Menge eines Gemischs von wenigstens einem Fluoreszenzfarbstoff gemäß Ansprüchen 1 bis 5 und wenigstens einem nichtfluoreszierenden Farbmittel in das Präparat.

18. Benzoxanthen der Formel

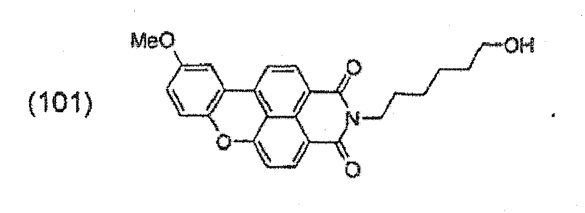

## Revendications

1. Composition cosmétique ou d'hygiène personnelle ayant une activité fluorescente, qui comprend au moins un colorant fluorescent choisi dans le groupe constitué par
a) les dérivés de pérylène de formules générales 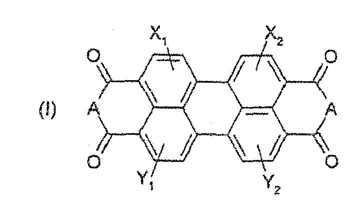 et 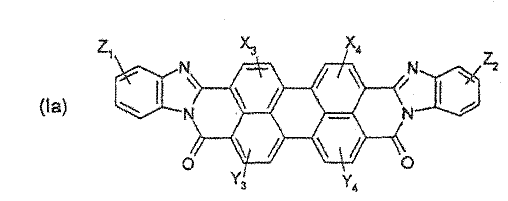 ou son régioisomère, dans lesquelles
A est 0, N-H, N-alkyle ou N-aryle et X₁, X₂, X₃, X₄, Y₁, Y₂, Y₃, Y₄, Z₁, Z₂ sont indépendamment un halogène, O - alkyle ou O-aryle ;
b) les fluorescéines de formule générale 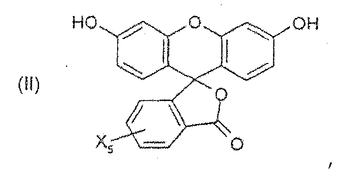 dans laquelle X₅ est Cl, Br ou I ;
c) les rhodamines de formule générale 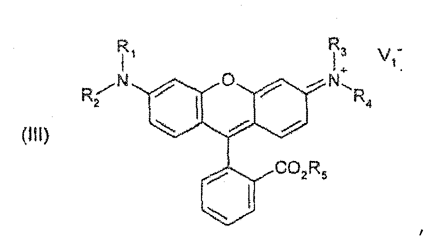 dans laquelle
les groupes R₁, R₂, R₃, R₄, R₅ sont indépendamment H ou un alkyle en C₁-C₁₂, et
V₁ est Cl, Br ou I ;
d) les pyronines de formule générale 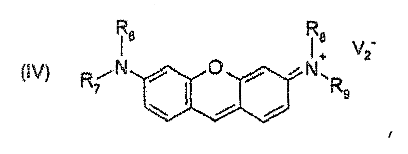 dans laquelle
les groupes R₆, R₇, R₈ et R₉ sont indépendamment H ou un alkyle en C₁-C₁₂, et
V₂ est Cl, Br ou I ;
e) les dérivés d'anhydride naphtalique de formule générale 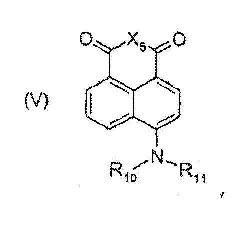 dans laquelle X₅ est O, N-H, N-alkyle ou N-aryle et R₁₀ et R₁₁ sont indépendamment H, alkyle ou aryle ;
f) les benzoxanthènes et les benzthioxanthènes de formules générales 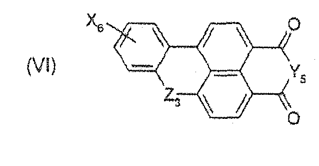 et 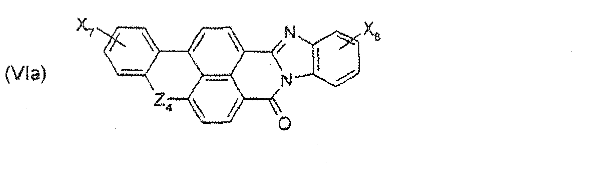 ou son régioisomère, dans lesquelles
X₆, X₇ et X₈ sont indépendamment O-alkyle, 0-aryle ou un halogène,
Y₅ est 0, N-H, N-alkyle ou N-aryle et Z₃ et Z₄ sont indépendamment O ou S ;
g) les dérivés de benzanthrone de formule générale 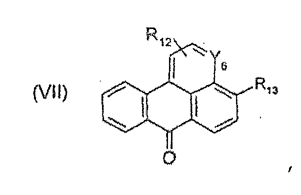 dans laquelle
Y₆ est C ou N et R₁₂ et R₁₃ sont indépendamment un halogène, alcoxy, amino ou alkylamino et, lorsque Y₆ est C, Y₆ plus R₁₃ peuvent former un cycle à 6 éléments, éventuellement condensé avec un groupe arylène ;
h) les coumarines de formule générale 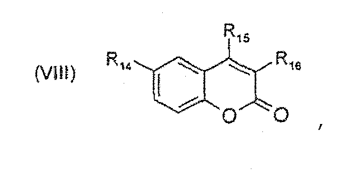 dans laquelle R₁₄ est H, alcoxy, amine ou alkylamino, et R₁₅ et R₁₆ sont indépendamment H, alkyle ou aryle ;
i) les dérivés d'isoindolo-[2,1-a]-benzimidazolone de formule générale 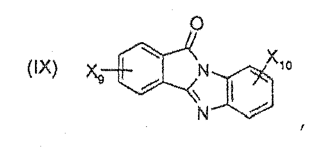 dans laquelle
X₉ et X₁₀ sont indépendamment l'hydrogène ou un halogène ;
j) les diphénylmaléimides de formule générale 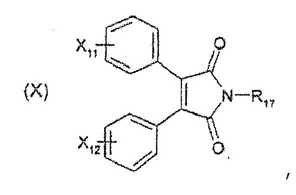 dans laquelle
R₁₇ est un alkyle ou aryle éventuellement substitué et X₁₁ et X₁₂ sont indépendamment H, alkyle, aryle, 0-alkyle, 0-aryle ou un halogène ;
k) les pigments azo de formule générale 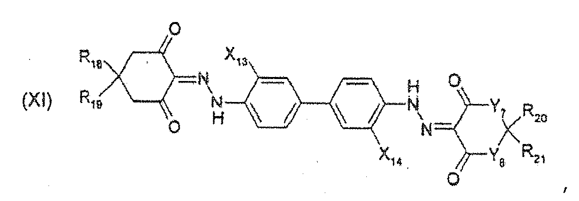 dans laquelle
X₁₃ et X₁₄ sont indépendamment un halogène, alcoxy, Y₇ et Y₈ sont indépendamment CH₂ ou 0 et R₁₈, R₁₉, R₂₀ et R₂₁ sont indépendamment un alkyle ou aryle éventuellement substitué ;
l) les dicétopyrrolo[3,4-c]pyrroles de formule générale 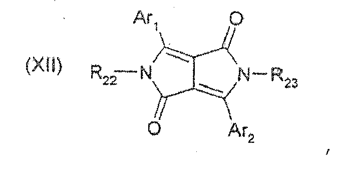 dans laquelle
Ar₁ et Ar₂ sont des radicaux aryle et R₂₂ et R₂₃ sont des radicaux alkyle ou aryle éventuellement substitués
et leurs mélanges et au moins un colorant non fluorescent.

2. Composition selon la revendication 1, qui confère une apparence de peau naturelle.

3. Composition selon la revendication 1, dans laquelle le colorant fluorescent a une teinte jaune et/ou rougeâtre.

4. Composition selon la revendication 1, dans laquelle le colorant fluorescent est choisi dans le groupe constitué par les dérivés de pérylène de formule (I) ou (Ia), les diphénylmaléimides de formule (X), les benzoxanthènes et benzthioxanthènes de formules (VI) et (VIa), les DPP N,N'-disubstitués de formule (XII) et l'isoindolo-[2,1-a]-benzimidazolone de
formule (IXa) 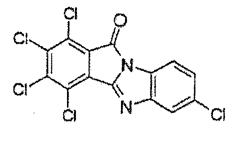 et leurs mélanges, les substituants dans les formules étant tels que définis dans la revendication 1.

5. Composition selon la revendication 1, dans laquelle le colorant fluorescent est le benzoxanthène de formule 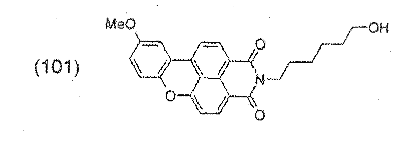 et ses mélanges.

6. Composition selon la revendication 1, dans laquelle le colorant non fluorescent est un colorant, un pigment ou une laque, ou un de leurs mélanges.

7. Préparation ou formulation cosmétique ou d'hygiène personnelle ayant une activité fluorescente, qui comprend 0,005 à 20 % en poids, par rapport au poids total de la préparation, d'un mélange d'au moins un colorant fluorescent selon les revendications 1 à 5 et d'au moins un colorant non fluorescent et d'un adjuvant cosmétiquement acceptable.

8. Préparation selon la revendication 7, qui comprend 0,005 à 2 % en poids du colorant fluorescent.

9. Préparation selon la revendication 7, qui comprend 0,005 à 20 % en poids du colorant non fluorescent.

10. Préparation selon la revendication 7, qui est sous la forme d'un rouge à lèvres, d'une préparation pour mascara, d'un maquillage pour les joues, d'un fard à paupières, d'un fond de teint, d'un eye-liner, d'une poudre pour le visage ou pour le corps ou d'un vernis à ongles.

11. Préparation selon la revendication 7, qui comprend des ingrédients actifs cosmétiquement acceptables supplémentaires.

12. Préparation selon la revendication 7, dans laquelle les ingrédients actifs cosmétiquement acceptables sont des glycols, des polyéthylène glycols, des polypropylène glycols, des monoalcanolamides, des charges polymères, inorganiques ou organiques non colorées, des conservateurs, des filtres et réflecteurs UV ou d'autres adjuvants et additifs couramment employés dans les produits cosmétiques.

13. Procédé de coloration d'une préparation cosmétique ou d'hygiène personnelle, qui comprend l'incorporation dans celle-ci d'une quantité tinctorialement efficace d'un mélange d'au moins un colorant fluorescent selon les revendications 1 à 5 et d'au moins un colorant non fluorescent selon la revendication 1.

14. Procédé selon la revendication 13, dans lequel le colorant fluorescent est le benzoxanthène de formule (101) et ses mélanges.

15. Procédé de coloration de la peau humaine, qui comprend l'application sur ladite peau d'une préparation cosmétique ou d'hygiène personnelle selon la revendication 7.

16. Procédé selon la revendication 15, dans lequel le colorant fluorescent est le benzoxanthène de formule (101) et ses mélanges.

17. Procédé de préparation d'une préparation cosmétique ou d'hygiène personnelle colorée selon la revendication 7, qui comprend l'incorporation dans ladite préparation d'une quantité tinctorialement efficace d'un mélange d'au moins un colorant fluorescent selon les revendications 1 à 5 et d'au moins un colorant non fluorescent.

18. Benzoxanthène de formule
